# EUROPEAN PATENT SPECIFICATION

(11) **EP 0 530 201 B1**
(45) Date of publication and mention of the grant of the patent: **03.08.1994**
(21) Application number: 91906711.6
(22) Date of filing: 15.03.1991
(51) Int. Cl.: A61M 25/00

(54) **A CATHETER LINER AND A METHOD OF MAKING THE SAME**
KATHETERBEKLEIDUNG UND VERFAHREN ZU IHRER HERSTELLUNG
REVETEMENT INTERIEUR DE CATHETER ET SON PROCEDE DE FABRICATION

(30) Priority: 15.03.1990 US 494649
(43) Date of publication of application: 10.03.1993
(73) Proprietor: W.L. GORE & ASSOCIATES, INC., Newark, Delaware 19714-9206 (US)
(72) Inventor: CRAWLEY, Jerald, Mathew, Flagstaff, AZ 86001 (US); DAVIDSON, Daniel, Francis, Flagstaff, AZ 86004 (US); MYERS, David, John, Camp Verde, AZ 86322 (US)
(74) Representative: Klunker . Schmitt-Nilson . Hirsch
(86) International application number: US9101771
(87) International publication number: WO9113648

(56) References cited:
- DE-B- 1 640 127
- FR-A- 1 591 251
- GB-A- 2 043 201
- SE-B- 315 449
- US-A- 4 106 509
- US-A- 4 280 500
- US-A- 4 430 083
- Patent Abstracts of Japan, vol 2, No 98, M 30, abstract of JP 53-67109, publ 1978-06-15 (NITTO DENKI KOGYO K.K.)

## Description

### FIELD OF THE INVENTION

This invention relates to a fluoropolymeric thinwall catheter liner of tape-wrapped construction, to catheters incorporating this liner, and to methods of producing such a fluoropolymeric thinwall catheter liner and catheters using the liner.

### BACKGROUND OF THE INVENTION

Fluoropolymers are preferred materials for medical catheters because they are biocompatible, inert, and lubricious. There is little risk of adverse biological or chemical reaction with fluoropolymer catheters. Their lubricious surfaces allow them to be used effectively as guiding catheters or used in conjunction with guidewire devices. Fluoropolymer catheters have less than ideal handling and structural characteristics. Relative to other available catheter materials, fluoropolymers offer relatively poor flexibility and kink resistance, poor crush resistance, inadequate column strength to allow the catheter to be pushed through complex passageways without buckling, and are liable to buckle when subjected to torsional forces.

Because of the compromises involved between the mechanical, biological and chemical requirements for catheter performance, many available "high performance" catheters are of composite construction that uses two or more different materials in order to take advantage of their different desirable properties. For example, a common composite construction involves the use of an outer polymeric jacket of, for example, polyethylene, an intermediate layer of a structural fiber such as braided stainless steel wire and an inner surface liner of a fluoropolymer such as fluorinated ethylene propylene (hereinafter FEP) or polytetrafluoroethylene (hereinafter PTFE). These composite catheters are able to offer an inert and lubricious inner fluoropolymeric surface, good column strength, crush resistance and torque resistance due to the intermediate structural fiber, and finally, good flexibility with a smooth outer surface due to the outer polymeric jacket.

This type of catheter construction is described by, for example, U.S. Patent 4,516,972.

One drawback to these composite construction catheters is their overall thickness. The outside diameter of a catheter is limited by the space available within the body passages that it is required to negotiate. The available inside diameter and consequent catheter volume is thus limited by the thickness of the composite construction that makes up the wall of the catheter.

As the purpose of the inner surface liner is to provide an inert and lubricious surface without any structural requirements other than maintaining an uninterrupted surface, the liner may functionally be very thin.

Fluoropolymer liners have heretofore been extruded with wall thickness down to about 0.038 mm (0.0015 inches).

### SUMMARY OF THE INVENTION

The present invention relates to a catheter liner comprised of a fluoropolymer tape wrapped around a mandrel to form a tube. The mandrel is subsequently removed from the inside of the liner. It also relates to catheters incorporating such a liner, and to methods of making the liner and catheters incorporating the liner.

### BRIEF DESCRIPTION OF THE DRAWINGS

FIGURE 1 shows a perspective view of a helically tape-wrapped fluoropolymer catheter liner of the present invention after the manufacturing mandrel has been removed.

FIGURE 2 shows a longitudinal section of a helically tape-wrapped fluoropolymer catheter liner of the present invention prior to the application of an outer jacket and prior to the removal of the manufacturing mandrel.

FIGURE 3 shows the construction of a finished catheter incorporating a helically tape-wrapped fluoropolymer catheter liner of the present invention, further having a first outer jacket of braided wire adjacent to and surrounding the liner and a second outer jacket of polyethylene adjacent to and surrounding the first jacket of braided wire.

FIGURE 4 shows a cross section of a longitudinally applied tape-wrap forming the fluoropolymer catheter liner of the present invention wherein the tape edges are abutted.

FIGURE 5 shows a cross section of a longitudinally applied tape-wrap forming the fluoropolymer catheter liner of the present invention wherein the tape edges are overlapped.

### DETAILED DESCRIPTION OF THE INVENTION

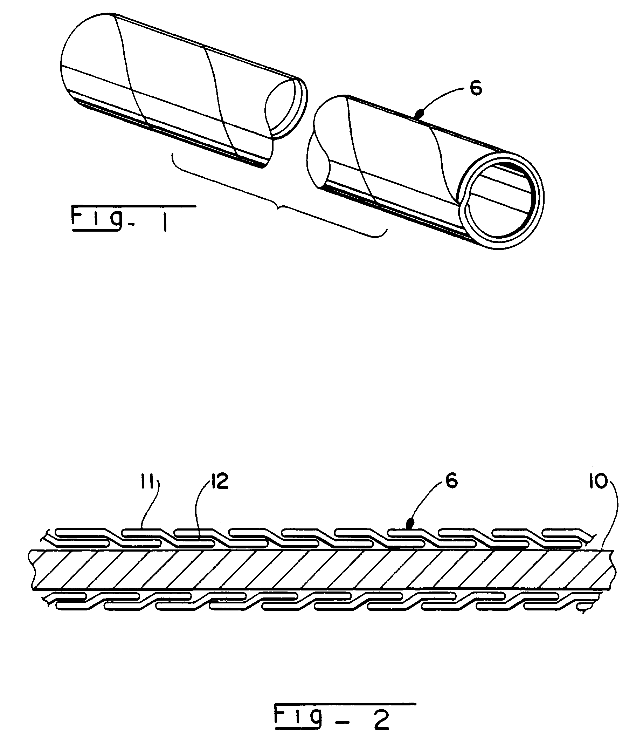

By wrapping a fluoropolymer tape around a mandrel to form a tube, it has been found possible to produce a fluoropolymer catheter liner having a thinner wall than previously available extruded fluoropolymer catheter liners. As shown in Figure 1, the catheter liner 6 of the present invention is made by diametrically slicing film rolls of wide, thin fluoropolymer film into rolls of narrow tape and wrapping the tape around a suitable mandrel. The tape may be wrapped around the mandrel either longitudinally, i.e., in cigarette wrap fashion, or helically. The tape edges may be abutted to adjacent tape edges or preferably adjacent tape edges may be overlapped. The wrapped tape is then heat-treated to melt-bond the edges of the tape to the adjacent layer of tape. The covering outer jacket or jackets may then be applied.

Figure 2 describes a longitudinal section of a catheter liner of the present invention prior to the application of an outer jacket and prior to removal of the manufacturing mandrel 10. In this configuration a helically applied tape wrapping is shown wherein half of the width of each tape layer 11 overlaps half of the width of the adjacent tape layer 12. In this fashion the liner is comprised of at least two layers of tape at any cross section and each tape edge is covered by the mid-portion of the width of the adjacent tape layer. It is apparent that more than two layers can be used if deemed necessary. It is also apparent that only one layer can provide a continuous fluoropolymer liner if the adjacent tape edges are sealed together by melt-bonding.

Figure 3 shows one possible catheter construction 7 using the catheter liner of the present invention. The tape-wrapped tubular liner 6 is shown having a braided first surrounding jacket of structural fibers 13, e.g., stainless steel wire or Nylon® fibers, and further having a second surrounding jacket of a polymeric material with a smooth outer surface, e.g., polyethylene.

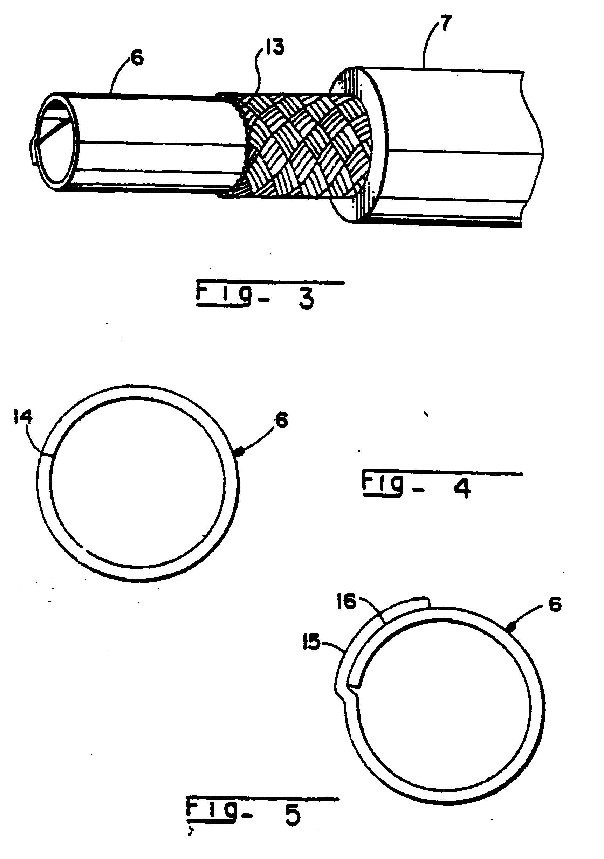

Figures 4 and 5 present different methods of finishing the tape edges. Figure 4 shows the tape edges 14 abutted while Figure 5 shows one tape edge 15 overlapping the second tape edge 16. Either abutted edges or overlapped edges may be used with helical and longitudinal wrapping techniques. Abutted edges offer the option of minimizing the wall thickness while overlapped edges offer the most secure bond. Melt-bonding together of adjacent edge surfaces is done thermally as previously described.

Fluoropolymer tape-wrapped catheter liners can also be made to serve as exterior-surface liners for any desired type of catheter simply by applying the tape to the outer surface of the catheter construction as applied around a mandrel to produce an inner liner.

Fluoropolymer tapes used herein include tapes of polytetrafluoroethylene, fluorinated ethylene propylene copolymer, perfluoro(alkyl vinyl ether) and ethylene/ fluoroethylene copolymers.

Such a fluoropolymeric catheter liner can be produced with PTFE film of 0.0013 mm (0.0005 inch) thickness, available in rolls from Norton Performance Plastics, Wayne, New Jersey. A roll may be sliced diametrically using a sharp blade while rotating the roll so that a narrow roll of tape is produced. In constructing the catheter of the following description, a tape of 12.7 mm (0.50 inch) width can be sliced from the roll of film. Using conventional tape-wrapping techniques, a length of this tape can be helically wrapped around a silver plated copper wire mandrel of 2.08 mm (0.082 inch) diameter. A slight amount of tension applied to the tape will allow it to lay uniformly and to fit tightly to the surface of the mandrel. The selected pitch results in the application of two layers of film over the surface of the mandrel. With a two-layer covering of film, all film edges are covered with an overlying or underlying layer of film as shown in Figure 2. The tape-wrapped mandrel can then be placed into an oven set at 380°C for a period sufficient to melt-bond the tape edges to the adjacent tape surfaces. This heat-treating process will result in the melt-bonding together of adjacent surfaces of the wrapped tape. The heat-treated tube, still on the wire mandrel, can then be immersed in a tank containing TETRA-ETCH® (W. L. Gore & Associates, Flagstaff, Arizona) for a period of about 15 seconds at about 25 degrees C. Residual TETRA-ETCH can then be removed in a rinse of isopropyl alcohol. The etching process will allow the subsequently applied layer of stainless steel wire braid to mechanically grip the outer surface of the tape-wrapped tube. This braid can be applied with a Steeger braiding machine using about 0.080 mm (40 gauge) stainless steel wire applied at about 11.8 picks per cm (30 picks per inch). Following the application of the braid, the assembly can be fed through a 25.4 mm (one inch) screw extruder where an outer jacket of 0.35 mm (0.014 inch) thick polyethylene can be applied. The catheter tubing comprising the tape-wrapped liner, braided wire jacket and extruded polyethylene outer jacket, all surrounding the wire mandrel, can then be cut into desired lengths (typically 1.4 m (4.5 feet)) using a wire cutting tool. The wire mandrel can then be extracted from the lumen of the catheter tubing by stripping about 5 cm (two inches) of the liner, braid and jacket from each end, gripping the exposed wire and applying tension to cause the wire to stretch. The stretch can cause a reduction in the diameter of the wire mandrel, freeing it from the surface of the catheter liner and allowing removal of the catheter tubing from the mandrel. Any desired catheter fittings or terminations may then be attached to the catheter tubing by existing techniques (e.g., injection molding or by the use of adhesives).

This process allows the manufacture of very long continuous lengths of catheter tubing which may subsequently be cut into any desired shorter length. The use of certain metallic mandrel surfaces during manufacture may leave toxic residues which must be removed, preferably by flushing the catheter liner surface with an appropriate solvent. Mandrels of other materials may also be used, e.g., polymeric mandrels of FEP or PTFE. The essential requirements of the mandrel are that it has a smooth exterior surface, that it is removable from the catheter liner surface after the catheter has been cut to length, and that any potentially harmful residue mandrel material is easily and entirely removable. The use of inert mandrel materials such as fluoropolymers is thus preferred. The mandrel may be of the form of a tube (hollow) or of a rod (solid).

### EXAMPLE 1

A 19 m (75 foot) length of fluoropolymeric catheter liner was made by helically tape-wrapping an FEP tape (from Norton Performance Plastics, Wayne, New Jersey) of 12.7 mm (0.5 inch) width and 0.015 mm (0.0006 inch) thickness about a mandrel of PTFE tubing (from Teleflex, Jaffrey, New Hampshire) of 1.96 mm (0.077 inch) outside diameter and about 19 m (75 foot) length. The tape was under tension during tape-wrapping, causing about 3% elongation and thinning of the tape to a thickness of about 0.0127 mm (0.0005 inch). The tape was applied with conventional tape-wrapping equipment to give a total wall thickness of two layers of tape at any cross section of the mandrel. The tape-wrapped mandrel was heat treated to melt-bond the tape edges in an oven set at 340°C for about 27 seconds, then etched, rinsed and braided according to the description of Example 1. A 61 cm (2 foot) length of FEP heat shrink tubing (SPC Technologies, Gaffney, North Carolina) was placed over a 61 cm (2 foot) length cut from the braided mandrel. The outside diameter of this tubing before shrinking was 6.35 mm (.250 inches), with a 0.25 mm (0.010 inch) thick wall. Heat shrinking was accomplished by pulling the tape-wrapped and braided mandrel with surrounding FEP tubing through a hollow, heated (340°C) die of 3.56 mm (0.140 inch) inside diameter and 25.4 mm (1.0 inch) length at a rate of about 30.5 cm (1 foot) per minute. The die was contained within a 15.2 cm (six inch) long oven set at 340°C that allowed pre-heating of the tubing before it contacted the die. In this manner the FEP heat shrink tubing formed the outer jacket of the catheter construction.

About 5 cm (two inches) of tape-wrapping, braid and jacket was stripped from each end of the mandrel. One exposed mandrel end was gripped between the jaws of a vise and tension was applied to the mandrel by pulling the other exposed mandrel end by hand. The tension caused elongation of the tubular PTFE mandrel, reducing its diameter and allowing the finished catheter comprised of the FEP tape-wrapped liner, the braided covering and surrounding outer FEP jacket to be removed from the mandrel without damage to the catheter.

## Claims

1. A catheter liner comprised of a fluoropolymer characterized in that a fluoropolymer tape (11) is wrapped into the form of a tube (6).

2. A catheter liner according to claim 1 wherein said tape (11) is helically applied.

3. A catheter liner according to claim 1 wherein said tape (11) is longitudinally applied.

4. A catheter liner according to claim 1 wherein said tape (11) is applied in at least two layers wherein one layer (12) overlaps the other layer (11).

5. A catheter liner according to claim 4 wherein said overlapping tape layers (11) and (12) are melt-bonded together.

6. A catheter liner according to claim 1 wherein said liner (6) has a wall thickness less than or equal to 0.038 mm (0.0015 inches).

7. A catheter liner according to claim 1 wherein said liner (6) has a wall thickness less than or equal to 0.025 mm (0.0010 inches).

8. A catheter tube comprised of a fluoropolymer tubular liner (6) and at least one outer tubular jacket (7) that concentrically surrounds said tubular liner (6), characterized in that the fluoropolymer tubular liner (6) is made from a fluoropolymer tape (11) wrapped into the form of a tube.

9. A catheter tube according to claim 8 wherein said outer jacket (7) is comprised of polyethylene.

10. A catheter tube according to claim 8 wherein said outer jacket (7) is comprised of braided stainless steel wire (13).

11. A catheter liner according to claim 1 wherein the fluoropolymer tube has a spiral seam.

12. A catheter liner according to claim 1 wherein the fluoropolymer tube has a longitudinal seam.

13. A catheter liner according to claim 11 or 12 wherein said tube (6) has a wall thickness less than or equal to 0.025 mm (0.0010 inch).

14. A method of making a fluoropolymeric catheter liner which comprises wrapping a fluoropolymer tape (11) around a mandrel (10) to form a tube (6).

15. The method of claim 14 wherein the tape (11) is helically wrapped around said mandrel.

16. The method of claim 14 wherein the tape (11) is longitudinally wrapped around said mandrel (10).

17. The method of claim 14 wherein said tape (11) is wrapped in overlapping layers, further comprising melt-bonding said overlapping layers together.

18. The method of claim 14 which further comprises removing the mandrel (10) from the catheter liner.

## Patentansprüche

1. Katheterfutter aus einem Fluorpolymer,
**dadurch gekennzeichnet,**
daß ein Fluorpolymer-Band (11) in der Form eines Rohres (6) gewickelt ist.

2. Katheterfutter nach Anspruch 1, bei dem das Band (11) schraubenförmig aufgebracht ist.

3. Katheterfutter nach Anspruch 1, bei dem das Band (11) in Längsrichtung aufgebracht ist.

4. Katheterfutter nach Anspruch 1, bei dem das Band (1) in mindestens zwei Schichten aufgebracht ist, wobei eine Schicht (12) die andere Schicht (11) überlappt.

5. Katheterfutter nach Anspruch 4, bei dem die überlappenden Bandschichten (11) und (12) durch Schmelzverbinden zusammengefügt sind.

6. Katheterfutter nach Anspruch 1, bei dem das Futter (6) eine Wandstärke von weniger oder gleich 0,038 mm (0,0015 Zoll) aufweist.

7. Katheterfutter nach Anspruch 1, bei dem das Futter (6) eine Wandstärke von weniger oder gleich 0,025 mm (0,0010 Zoll) aufweist.

8. Katheterrohr, bestehend aus einem rohrförmigen Fluorpolymer-Futter (6) und mindestens einem äußeren rohrförmigen Mantel (7), welcher das rohrförmige Futter (6) konzentrisch umgibt,
**dadurch** **gekennzeichnet**,
daß das rohrförmige Fluorpolymer-Futter (6) aus einem Fluorpolymer-Band (11) gefertigt ist, welches in Form eines Rohres gewickelt ist.

9. Katheterrohr nach Anspruch 8, bei dem der äußere Mantel (7) aus Polyethylen besteht.

10. Katheterrohr nach Anspruch 8, bei dem der äußere Mantel (7) aus geflochtenem Draht aus rostfreiem Stahl (13) besteht.

11. Katheterfutter nach Anspruch 1, bei dem das Fluorpolymerrohr eine spiralförmige Naht besitzt.

12. Katheterfutter nach Anspruch 1, bei dem das Fluorpolymer-Rohr eine Längsnaht besitzt.

13. Katheterfutter nach Anspruch 11 oder 12, bei dem das Rohr (6) eine Wanddicke von weniger oder gleich 0,025 mm (0,0010 Zoll) aufweist.

14. Verfahren zum Herstellen eines Fluorpolymer-Katheterfutters, bei dem um einen Dorn (10) ein Fluorpolymer-Band (11) in Form eines Rohres (6) gewickelt wird.

15. Verfahren nach Anspruch 14, bei dem das Band (11) schraubenförmig um den Dorn gewickelt wird.

16. Verfahren nach Anspruch 14, bei dem das Band (11) in Längsrichtung um den Dorn (10) gewickelt wird.

17. Verfahren nach Anspruch 14, bei dem das Band (11) in einander überlappenden Schichten gewickelt wird, wobei außerdem die einander überlappenden Schichten einer Schmelzverbindung unterzogen werden.

18. Verfahren nach Anspruch 14, bei dem der Dorn (10) aus dem Katheterfutter entfernt wird.

## Revendications

1. Revêtement de cathéter constitué de polymère fluoré, caractérisé en ce qu'on enroule un ruban de polymère fluoré (11) en forme de tube (6).

2. Revêtement de cathéter selon la revendication 1, dans lequel ledit ruban (11) est déposé en hélice.

3. Revêtement de cathéter selon la revendication 1, dans lequel ledit ruban (11) est déposé longitudinalement.

4. Revêtement de cathéter selon la revendication 1, dans lequel ledit ruban (11) est déposé sur au moins deux couches parmi lesquelles une couche (12) recouvre l'autre couche (11).

5. Revêtement de cathéter selon la revendication 4, dans lequel lesdites couches de ruban en recouvrement (11) et (12) sont soudées ensemble par fusion.

6. Revêtement de cathéter selon la revendication 1, dans lequel ledit revêtement (6) a une épaisseur de paroi inférieure ou égale à 0,038 mm (0,0015 pouce).

7. Revêtement de cathéter selon la revendication 1, dans lequel ledit revêtement (6) a une épaisseur de paroi inférieure ou égale à 0,025 mm (0,0010 pouce).

8. Tube cathéter comprenant un revêtement tubulaire en polymère fluoré (6) et au moins une enveloppe tubulaire externe (7) qui entoure de façon concentrique ledit revêtement tubulaire (6), caractérisé en ce que le revêtement tubulaire en polymère fluoré (6) est un ruban de polymère fluoré (11) enroulé sous forme de tube.

9. Tube cathéter selon la revendication 8, dans lequel ladite enveloppe externe (7) est en polyéthylène.

10. Tube cathéter selon la revendication 8, dans lequel ladite enveloppe externe (7) est en fil d'acier inoxydable tressé (13).

11. Revêtement de cathéter selon la revendication 1, dans lequel le tube de polymère fluoré a une ligne de soudure en spirale.

12. Revêtement de cathéter selon la revendication 1, dans lequel le tube de polymère fluoré a une ligne de soudure longitudinale.

13. Revêtement de cathéter selon la revendication 11 ou 12, dans lequel ledit tube (6) a une épaisseur de paroi inférieure ou égale à 0,025 mm (0,0010 pouce).

14. Procédé de fabrication d'un revêtement de cathéter en polymère fluoré qui comprend d'enrouler un ruban de polymère fluoré (11) sur un mandrin (10) pour former un tube (6).

15. Procédé selon la revendication 14, dans lequel le ruban (11) est enroulé en hélice sur ledit mandrin.

16. Procédé selon la revendication 14, dans lequel le ruban (11) est enroulé longitudinalement autour dudit mandrin (10).

17. Procédé selon la revendication 14, dans lequel ledit ruban (11) est enroulé en couches superposées, qui comprend en outre de souder ensemble par fusion lesdites couches superposées.

18. Procédé selon la revendication 14 qui comprend en outre d'éliminer le mandrin (10) du revêtement de cathéter.
